# EUROPEAN PATENT SPECIFICATION

(11) **EP 1 651 273 B1**
(45) Date of publication and mention of the grant of the patent: **29.08.2012**
(21) Application number: 04777860.0
(22) Date of filing: 08.07.2004
(51) Int. Cl.: A61K 47/34, A61K 9/00, A61K 9/16, A61K 9/70

(54) **POLY-4-HYDROXYBUTYRATE MATRICES FOR SUSTAINED DRUG DELIVERY**
POLY-4-HYDROXYBUTYRAT-MATRIZES ZUR KONTINUIERLICHEN ARZNEISTOFFZUFÜHRUNG
MATRICES POLY-4-HYDROXYBUTYRATE POUR UNE ADMINISTRATION PROLONGEE DE MEDICAMENTS

(30) Priority: 08.07.2003 US 485373 P; 30.07.2003 US 491430 P
(43) Date of publication of application: 03.05.2006
(73) Proprietor: Tepha, Inc., Lexington MA 02421 (US)
(72) Inventor: HASIRCI, Vasif, N., 06530 Ankara (TR); KESKIN, Dilek, 06660 Ankara (TR)
(74) Representative: Wright, Andrew John
(86) International application number: PCT/US2004/022040
(87) International publication number: WO 2005/007195

(56) References cited:
- EP-A- 0 945 137
- US-B1- 6 548 569
- TÜRESIN F ET AL: "Biodegradable polyhydroxyalkanoate implants for osteomyelitis therapy: in vitro antibiotic release." JOURNAL OF BIOMATERIALS SCIENCE. POLYMER EDITION. 2001, vol. 12, no. 2, 2001, pages 195-207, XP009040103 ISSN: 0920-5063 cited in the application
- PATENT ABSTRACTS OF JAPAN vol. 1996, no. 10, 31 October 1996 (1996-10-31) & JP 8 151321 A (TAKEDA CHEM IND LTD), 11 June 1996 (1996-06-11)

## Description

### Background of the Invention

The present invention generally relates to drug delivery systems , derived from poly-4-hydroxybutyrate.

The use of biodegradable polymers to make drug delivery systems is well established. For example, Takeda Pharmaceuticals has developed a formulation based on polylactide-co-glycolide for the delivery of LHRH (leuteinizing hormone-releasing hormone) that can be administered monthly and provide a prolonged therapeutic level of LHRH for the treatment of prostate cancer, and Guilford Pharmaceuticals has developed a formulation of the cancer chemotherapeutic drug, carmustine (BCNU), sold under the tradename of GLIADEL™, which is based on a degradable polyanhydride polymer. Another company, Atrix Laboratories has developed a system called ATRIDOX™ based on a degradable polylactide (PLA) for the delivery of the antibiotic doxycycline for periodontal therapy. Despite these positive developments there still exists a need to develop new and improved drug delivery systems. Devices based on PLA, for example, have been reported to cause localized inflammation (see U.S Patent No. 6,214,387 to Berde and Langer). Berde, et al. (Abstracts of Scientific Papers, 1990 Annual Meeting, Amber. Soc. Anesthesiologists, 73:A776, September 1990) have also reported drawbacks of certain degradable polyanhydride drug delivery systems that include fast initial release of drug, and inflammatory responses to the device or the formation of a capsule of serous material or fibrin. For certain applications, such as the treatment of chronic or persistent pain, longterm contraception or administration of antibiotics, growth factors, or chemotherapeutics, or prevention of restenosis after stent implantation, it would be advantageous to develop systems that can administer drugs for prolonged periods of time. It would also be desirable to develop systems that can be loaded with large amounts of drug to provide prolonged release' and/or to permit the use of smaller devices, as well as systems that can deliver different types of drugs (e.g. hydrophobic, hydrophilic, peptides, proteins, DNA and RNA) without decreasing the activity (for example, resulting from the unfolding of an active peptide or protein) of the drug.

Accordingly, it is the object of this invention to provide an improved biodegradable controlled release system that administers a drug for a prolonged period of time.

It is a further object of this invention to provide an improved biodegradable controlled release system that can be loaded with large amounts of drug.

It is yet another object of this invention to provide methods for preparing and modulating the rate of release of the drug from the controlled release system.

### Summary of the Invention.

Biodegradable controlled release systems providing prolonged controlled release of drugs, and methods for the manufacture thereof are disclosed. The systems are formed from a biocompatible, biodegradable polymer, in particular poly-4-hydroxybutyrate (PHA440). Drugs are generally incorporated into the polymer using a method that yields a uniform dispersion. The type of drug and the quantity are selected based on the known pharmaceutical properties of these compounds. The systems may be administered for example by implantation, injection, topical Administration, or oral ingestion. They may also be used in combination with a medical device, for example, a stent. A major advantage of the drug delivery system is that it does not need to be removed after use since it is slowly degraded and cleared by the patient's body. The device has desirable physical properties, including strength, modulus and elongation.

### Brief Description of the Drawings

Figures 1A and 1B are the chemical structures of poly-4-hydroxybutyrate (PHA4400) and poly-3-hydroxybutyrate-co-4-hydroxybutyrate (PHA3444).
Figure 2 is sdescription of the biosynthetic pathways for the production of PHA4400 (P4HB). Pathway enzymes are: 1. Succinic semialdehyde dehydrogenase, 2. 4-hydroxybutyrate dehydrogenase, 3. diol oxidoreductase, 4. aldehyde dehydrogenase, and 5. Coenzyme A transferase and 6. PHA synthetase.
Figures 3A and 3B are graphs of the release of Tetracycline from PHA4400:TC (2:1) rods (37°C, PBS, 364.0 nm) (n=4). Figure 3A, Average cumalative (%) release vs. time; Figure 3B, Average Cum. (%) release vs. square root time.
Figures 4A and B are graphs of release of Tetracycline (neutral) from PHA4400:TCN (2:1) rods (37°C, PBS, 357.6 nm) (n=4). Figure 4A, Average Cum. (%) release vs. time; Figure 4B, Average Cumulative (%) release vs. square root time.
Figures 5A and 5B are graphs of the release of Tetracycline from PHA3444-34%:TC (2:1) rods (37°C, PBS, 364.0 nm) (n=4). Figure 5A shows Average Cumulative (%) release versus time; Figure 5B shows Average Cumulative (%) release versus square root time.
Figures 6A and 6B are graphs of the release of Tetracycline Neutral (TCN) from PHA3444-34%:TCN (2:1) rods (37°C, PBS, 364.0 nm) (n=4). Figure 6A shows Average Cumulative (%) release versus time; Figure 6B shows Average Cumulative (%) release versus square root time.

### Detailed Description of the Invention

Biodegradable drug delivery systems for the controlled and prolonged release of drugs are provided. These systems can be used where it is necessary to administer a controlled amount of drug over a prolonged period, and/or to employ a system requiring a high drug loading.

### I. Definitions

Poly-4-hydroxybutyrate means a homopolymer comprising 4-hydroxybutyrate units. It may be referred to as PHA4400 or P4HB. Copolymers of poly-4-hydroxybutyrate mean any polymer comprising 4-hydroxybutyrate with one or more different hydroxy acid units, for example, poly-3-hydroxybutyrate-co-4-hydroxybutyrate (PH3444).

Biocompatible refers to materials that are not toxic, and do not elicit severe inflammatory or chronic responses *in vivo.* Any metabolites of these materials should also be biocompatible.

Biodegradation means that the polymer must break down or dissolve away *in vivo*, preferably in less than two years, and more preferably in less than one year. Biodegradation refers to a process in an animal or human. The polymer may break down by surface erosion, bulk erosion, hydrolysis or a combination of these mechanisms.

The term "microspheres" also includes nanospheres, microparticles, and microcapsules.

### II. Drug Delivery Devices

### A. Polymers

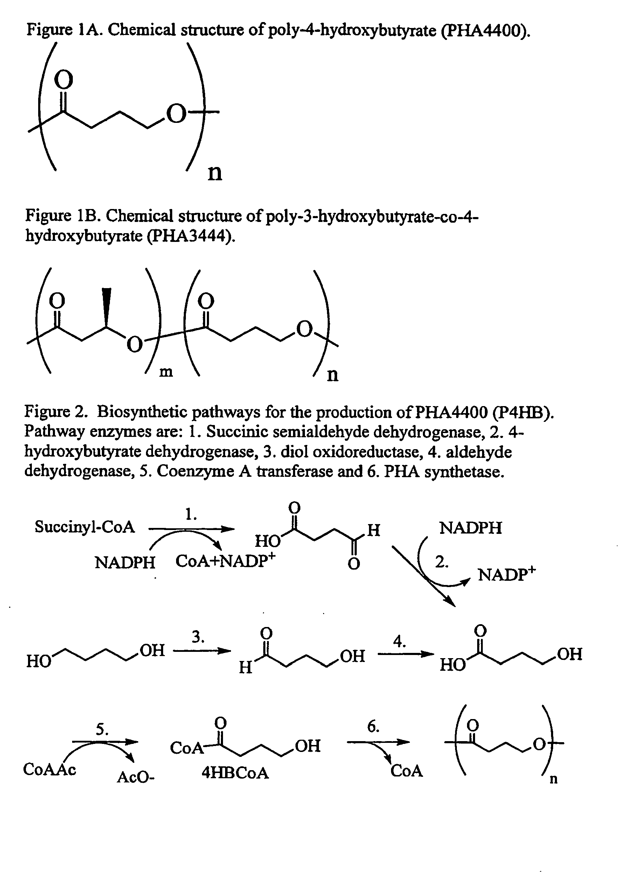

Poly-4-hydroxybutyrate (PHA4400) is a strong, pliable thermoplastic that is produced by a fermentation process (see US Patent No. 6,548,569 to Williams et al.). Despite its biosynthetic route, the structure of the polyester is relatively simple (Figure 1A). The polymer belongs to a larger class of materials called polyhydroxyalkanoates (PHAs) that are produced by numerous microorganims (for reviews see: Steinbüchel, A. (1991) Polyhydroxyalkanoic acids, in Biomaterials, (Byrom, D., Ed.), pp. 123-213. New York: Stockton Press; Steinbüchel, A. and Valentin, H.E. (1995) FEMS Microbial. Lett. 128:219-228; and Doi, Y. (1990) Microbial Polyesters, New York: VCH).

Polyhydroxyalkanoates (PHAs) are a class of naturally occurring polyesters that are synthesized by numerous organisms in response to environmental stress. For reviews, see Byrom, 3Miscellaneous Biomaterials,2 in Byrom, ed., Biomaterials MacMillan Publishers, London, 1991, pp. 333-59; Hocking & Marchessault, 3Biopolyesters2 in Griffin, ed., Chemistry and Technology of Biodegradable Polymers, Chapman and Hall, London, 1994, pp.48-96; Holmes, 3Biologically Produced (R)-3-hydroxyalkanoate Polymers and Copolymers2 in Bassett, ed., Developments in Crystalline Polymers, Elsevier, London, vol. 2, 1988, pp. 1-65; Lafferty et al., 3microbial Production of Poly-β-hydroxybutyric acid2 in Rehm & Reed, eds., Biotechnology, Verlagsgesellschaft, Weinheim, vol. 66, 1988, pp. 135-76; Müller & Seebach, Angew. Chem. Int. Ed. Engl. 32:477-502 (1993); Steinbüchel, 3Polyhydroxyalkanoic Acids2 in Byrom, ed., Biomaterials, MacMillan Publishers, London, 1991, pp. 123-213; Williams & Peoples, CHEMTECH, 26:38-44, (1996), and the recent review by Madison & Husiman, Microbiol. & Mol. Biol. Rev. 63:21-53 (1999).

The PHA biopolymers may be broadly divided into three groups according to the length of their pendant groups and their respective biosynthetic pathways. Those with short pendant groups, such as polyhydroxybutyrate (PHB), a homopolymer of R-3-hydroxybutyric acid (R-3HB) units, are highly crystalline thermoplastic materials, and have been known the longest (Lemoigne & Roukhelman, Annales des fermentations, 5:527-36 (1925)). A second group of PHAs containing the short R-3HB units randomly polymerized with much longer pendant group hydroxy acid units were first reported in the early seventies (Wallen & Rohwedder, Environ. Sci. Technol., 8:576-79 (1974)). A number of microorganisms which specifically produce copolymers of R-3HB with these longer pendant group hydroxy acid units are also known and belong to this second group (Steinbüchel & Wiese, Appl. Microbiol. Biotechnol., 37:691-97 (1992)). In the early eighties, a research group in The Netherlands identified a third group of PHAs, which contained predominantly longer pendant group hydroxy acids (De Smet, et al., J. Bacteriol., 154:870-78 (1983)).

The PHA polymers may constitute up to 90% of the dry cell weight of bacteria, and are found as discrete granules inside the bacterial cells. These PHA granules accumulate in response to nutrient limitation and serve as carbon and energy reserve materials. Distinct pathways are used by microorganisms to produce each group of these polymers. One of these pathways leading to the short pendant group polyhydroxyalkanoates (SPGPHAs) involves three enzymes, namely thiolase, reductase and PHB synthase (sometimes called polymerase). Using this pathway, the homopolymer PHB is synthesized by condensation of two molecules of acetyl-Coenzyme A to give acetoacetyl-Coenzyme A, followed by reduction of this intermediate to R-3-hydroxybutyryl-Coenzyme A, and subsequent polymerization. The last enzyme in this pathway, namely the synthase, has a substrate specificity that can accommodate C3-C5 monomeric units including R-4-hydroxy acid and R-5-hydroxy acid units. This biosynthetic pathway is found, for example, in the bacteria *Zoogloea ramigera* and *Alcaligenes eutrophus*.

The biosynthetic pathway which is used to make the third group of PHAs, namely the long pendant group polyhydroxyalkanoates (LPGPHAs), is still partly unknown, however, it is currently thought that the monomeric hydroxyacyl units leading to the LPGPHAs are derived by the β-oxidation of fatty acids and the fatty acid pathway. The R-3-hydroxyacyl-Coenzyme substrates resulting from these routes are then polymerized by PHA synthases (sometimes called polymerases) that have substrate specificities favoring the larger monomeric units in the C6-C 14 range. Long pendant group PHAs are produced, for example, by *Pseudomonads.*

Presumably, the second group of PHAs containing both short R-3HB units and longer pendant group monomers utilize both the pathways described above to provide the hydroxy acid monomers. The latter are then polymerized by PHA synthases able to accept these units.

In all about 100 different types of hydroxy acids have been incorporated into PHAs by fermentation methods so far (Williams, et. al., Int. J. Biol. Macromol., 25:111-21 (1999)). Notably, these include PHAs containing functionalized pendant groups such as esters, double bonds, alkoxy, aromatic, halogens and hydroxy groups.

During the mid-1980¹s, several research groups were actively identifying and isolating the genes and gene products responsible for PHA synthesis. These efforts have lead to the development of transgenic systems for production of PHAs in both microorganism and plants, as well as enzymatic methods for PHA synthesis. Such routes could increase further the available PHA types. These advances have been reviewed in Williams & Peoples, *CHEMTECH,* 26:38-44 (1996), Madison & Huisman, *Microbio*/*. Mol. Biol. Rev*., 63:21-53 (1999), and Williams & Peoples, *Chem. Br.* 33:29-32 (1997).

In addition to using biological routes for PHA synthesis, PHA polymers may also be derived by chemical synthesis. One widely used approach involves the ring-opening polymerization of ß-lactone monomers using various catalysts or initiators such as aluminoxanes, distannoxanes, or alkoxy-zinc and alkoxy-aluminum compounds (see Agostini, et al., Polym. Sci., Part A-1, 9:2775-87 (1971); Gross, et al., Macromolecules, 21:2657-68 (1988); Dubois, et al., Macromolecules, 26:4407-12 (1993); Le Borgne & Spassky, Polymer, 30:2312-19 (1989); Tanahashi & Doi, Macromolecules, 24:5732-33 (1991); Hori, et al., Macromolecules, 26:4388-90 (1993); Kemnitzer, et al., Macromolecules, 26:1221-29 (1993); Hori, et al., Macromolecules, 26:5533-34 (1993); Hoking & Marchessault; Polym. Bull., 30:163-70 (1993); U.S. Patent Nos. 5,489,470 and 5,502,116 to Noda). A second approach involves condensation polymerization of esters and is described in U.S. Pat. No. 5,563,239 to Hubbs, et al., and references therein. Researchers also have developed chemo-enzymatic methods to prepare PHAs. Xie et at., Macromolecules, 30:6997-98 (1997), for example, discloses a ring opening polymerization of beta-butyrolactone by thermophilic lipases to yield PHB.

Several biosynthetic routes are currently known to produce PHA4400, and these are shown in Figure 2. (Chemical synthesis of PHA4400 has been attempted, but it has been impossible to produce the polymer with a sufficiently high molecular weight necessary for most applications, see Hori, et al (1995) Polymer 36:4703-4705).

Tepha, Inc. (Cambridge, MA) produces PHA4400 and PHA 3444 for the development of medical uses, and has filed separate Device Master Files with the United States Food and Drug Administration (FDA) for PHA4400 and PHA3444. Methods to control molecular weight of PHA polymers have been disclosed by US Patent No. 5,811,272 to Snell et al., and methods to purify PHA polymers for medical use have been disclosed by U.S Patent No. 6,245,537 to Williams et al. PHAs with degradation rates *in vivo.* of less than one year have been disclosed by US Patent No. 6,548,569 to Williams et al. and PCT WO 99/32536 to Martin al.

The use of PHAs to produce a range of medical devices has been disclosed. For example, US Patent No. 6,514,515 to Williams discloses tissue engineering scaffolds, US Patent No. 6,555,123 to Williams and Martin discloses soft tissue repair, augmentation and viscosupplementation, PCT WO 01/15671 to Williams discloses flushable disposable polymeric products, and PCT WO 01/19361 to Williams and Martin discloses PHA prodrug therapeutic compositions. Other applications of PHAs have been reviewed by Williams, S.F. and Martin, D.P. (2002) Applications of PHAs in medicine and pharmacy, in Biopolymers: Polyesters, III (Doi, Y. and Steinbuchel, A., Eds.) vol. 4, pp. 91-127. Weinheim: Wiley-VCH.

Several reports have described the use of copolymers of 4-hydroxybutyrate with 3-hydroxybutyrate (PHA3444) to develop drug delivery systems. For example, Gürsel, et al. (2001) Biomaterials 22:73-80, Korkusuz, et al. (2001) J. Biomed. Mater. Res. 55:2117-228, and Türesin et al. (2001) J. Biomater. Sci. Polymer Edn. 12:195-207 have described the use of PHA3444 to develop controlled release systems for the treatment of osteomyelitis. U.S. Patent No. 6,548,569 to williams et al. discloses different forms of PHA4400 (also known as P4HB) including compression molded porous samples, fibers, foams, coated meshes, and microspheres.

The polyhydroxyalkahoate polymers should be biocompatible and biodegradable. The polymers are typically prepared by fermentation. Preferred polymers are poly-4-hydroxybutyrate. Examples of these polymers are produced by Tepha, Inc. of Cambridge, MA using transgenic fermentation methods, and have weight average molecular weighs in the region of 50,000 to 1,000,000.

### B. Drugs

The drug used in a particular drug release formulation will depend upon the specific treatment. The examples describe antibiotics for treatment or prevention of infection, however, the utility of the polymers shown here are not limited to the use of antibiotics. Other drugs that could be potentially used in a drug release formulation from the polymers described here include medicine for the treatment of disease, injury or pain The drug can be a protein, peptide, polysaccharide, nucleic acid molecule, or synthetic or natural organic compound. These include but are not limited to bioactive peptides or proteins, such as growth factors, hormones, and cell attachment factors, anti-proliferative agents, antibiotics, chemotherapeutics, anesthetics, small drug molecules, steroids, enzymes, lipids, antigens, antibodies, surfactants, vitamins, flavoring agents, radioactive molecules, sweeteners, nutritional agents, and fragrances.

The percentage loading of the drug will also depend on the specific treatment and the desired release kinetics. The polymers are suitable for drug loadings to at least 33% (i.e. polymer to drug ratios of 2:1). When the PHA polymers described here are loaded with drug (2:1), the drug release formulations remained flexibly and retained good mechanical properties. Higher loadings of up to 1:1 also can be used and show good mechanical properties. The desired release kinetics will also depend upon the specific treatment. In a preferred embodiment, the device is characterized by linear or zero-order drug release. In a more preferred embodiment, the device does not release a burst of the drug. Drug will typically be released over a period of at least 21 days, at least one month, at least three months, or at least six months. In general a linear release of drug is preferred. The length of time for the drug release can be controlled by selection of the drug, varying the drug loading and the shape and configuration of the drug release device. The examples show nearly linear release of the antibiotic drugs over a period of 18 days. It is expected that the period of release will extend beyond this time period and can be varied by the device configuration.

### III. Method of Manufacture

The drug delivery systems are preferably manufactured by a method that evenly disperses the drug throughout the device, such as solvent casting, spray drying, and melt extrusion. They may also, however, be prepared by other methods such as compression molding and lyophilization. The delivery systems may take virtually any form, including granules, sheets, films, and particles, such as microspheres, nanospheres, microparticles, and microcapsules, as well as molded forms, such as patches, tablets, suspensions, pastes, rods, disks, pellets, and other molded forms. Preferred devices include microspheres and implantable molded devices. Desired release profiles may be further tailored by altering the physical shape of the delivery system. (For example, by altering the surface area or porosity of the device, or by varying the polymer to drug ratio.) Other components may also be introduced into the formulation as necessary to aid or improve delivery, administration, drug release, and/or monitoring.

### IV. Method of Administration

The method of administration of the drug delivery system will be dependent upon the type of drug and its known pharmaceutical properties, and the form of the delivery system. Small devices may be implanted, microspheres may be injected, patches affixed to the skin, and tablets, suspension, and capsules taken orally. Preferred methods of administration are by injection and implantation.

As demonstrated by the examples, these polymers are particularly useful for construction of drug release systems with controllable rates. They are also suitable for loading significantly larger quantities of drug within a typical controlled release sample.

Non-limiting examples are given herein to describe the methods for preparing the drug delivery systems, and to illustrate the prolonged drug release profile and high drug loadings that can be achieved.

### EXAMPLE 1: PHA4400 Rod Preparation

PHA4400 powder (Tepha, Inc., Cambridge, MA) (Mw ~450 K) was weighed, placed in liquid nitrogen to render it brittle, and ground three times in a blender for 5 s duration. Chloroform was added to the resulting granules until a paste was formed, and then an antibiotic drug was added in a 2:1 ratio of polymer:drug by weight. The paste was then introduced into a mold measuring 150x5x5 mm, and left to dry at ambient temperature. The dry molded formulation was removed from the mold, and sections 2 mm thick were cut yielding rods with approximate dimensions of 2x5x5 mm.

Rod samples containing two different forms of tetracycline antibiotic were prepared. These were a highly water soluble HCl form, designated TC, and a neutral form, designated TCN (FAKO Pharmaceutical Co., Istanbul). Extinction coefficients for these two forms were determined as 0.117 (µg/mL⁻) at 364 nm for TC and 0.145 (µg/mL)⁻¹ at 357.6 nm for TCN at 37°C. Rods containing 10:1 and 5:1 ratios of PHA4400 to drug were also prepared as described above.

### EXAMPLE 2: Drug Release from PHA4400 Rods

A rod prepared as described in Example 1 was pre-weighed and introduced into a 50 mL Falcon tube containing 30 mL of 0.1 M pH 7.4.PBS (phosphate buffer). The tube was placed in a shaking water bath and maintained at 37 °C. Release of the antibiotic was determined by UV spectrophotometry using the extinction coefficients cited in Example 1 at 4 hours, 24 hours, and then daily with complete replacement of the release buffer with PBS. The release studies where carried out in a minimum of triplicate for each antibiotic.

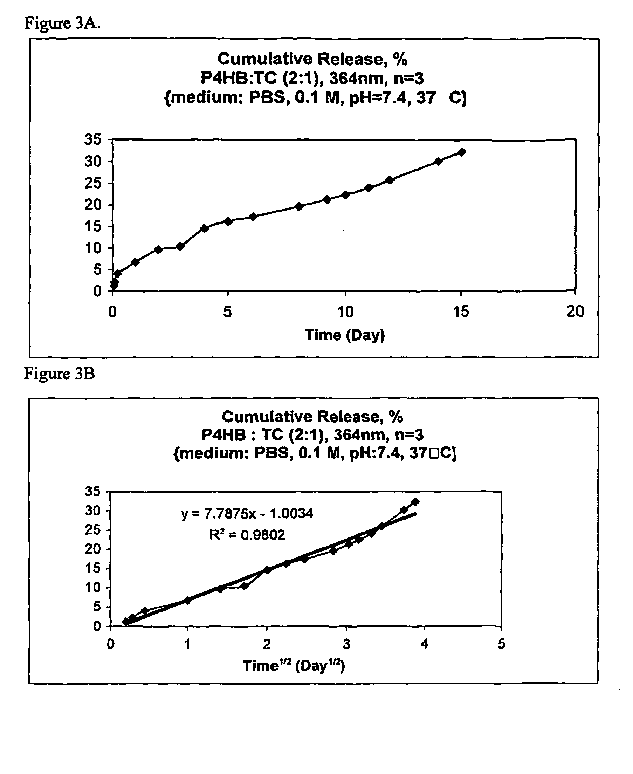

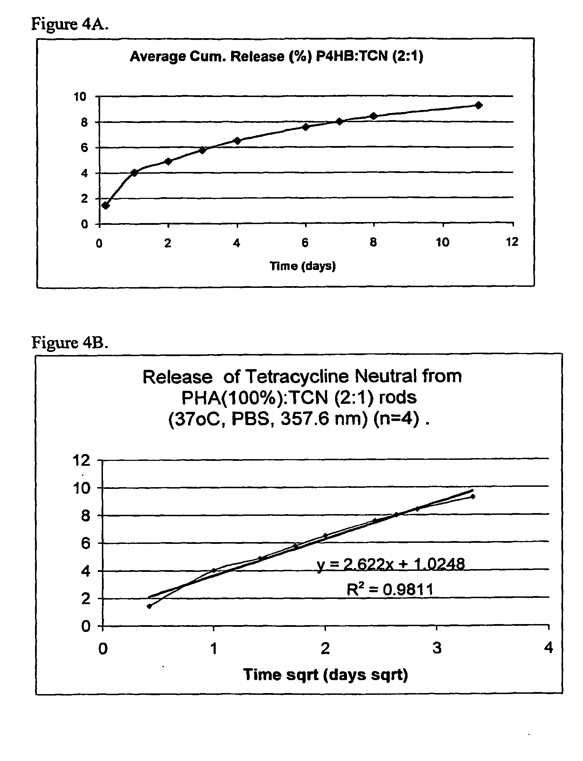

The release behavior appeared to follow Higuchi release kinetics (the k values for TC and TCN were 7.79 and 2.62, respectively) for an 11 -day period releasing only a fraction of the total content, see Figures 3 and 4. TC released at a higher rate than the less waster soluble TCN. The average cumulative release of TC at 11 days was approximately 25% versus 9% for TCN, demonstrating long term or sustained release.

Release from polymer loaded 10:1 was also determined. Release PHA4400 loaded 10:1 with TC showed zero order release over a period of about 15 days, with a minor short burst initially, possibly due to remnants of drug crystals left on the surface during drying. Release of TCN showed no burst, and almost perfect zero order release after the first hour, with a total of 17% in fifteen days, indicating that drug release should continue for several months.

Release from polymer loaded 5:1 was similar, with a slighter higher level of release and shorter duration compared to the 101:1 loaded system.

### Reference EXAMPLE 3: PHA3444 Rod Preparation

PHA3444 (34% 44) powder (Tepha, Inc., Cambridge, MA) (Mw ~477 K) was weighed, placed in liquid nitrogen to render it brittle, and ground three times in a blender for 5 s duration Chloroform was added to the , resulting granules until a paste was formed, and then an antibiotic drug was added in a 2:1 ratio of polymer:drug by weight The paste was then introduced into a mold measuring 150x5x5 mm, and left to dry at ambient temperature. The dry molded formulation was removed from the mold, and section 2 mn thick were cut yielding rods with approximate dimensions of 2x5x5 mm (as in Example 2).

Rod samples containing two different forms of tetracycline antibiotic were prepared. These were a highly water soluble HCl form, designated TC, and a neutral form, designated TCN (as above).

### Reference EXAMPLE 4: Drug Release from PHA3444-34% Rods

A rod prepared as described in Example 3 loaded 2: 1 with TC or TCN was pre-weighed and introduced into a 50 mL Falcon tube containing 30 mL of 0.1 M pH 7.4 PBS (phosphate buffer). The tube was placed in a shaking water bath and maintained at 37 °C. Release of the antibiotic was determined by UV spectrophotometry using the extinction coefficients cited in Example 1 at 4 hours, 24 hours, and then daily with complete replacement of the release buffer with PBS. The release studies were carried out in a minimum of triplicate for each antibiotic.

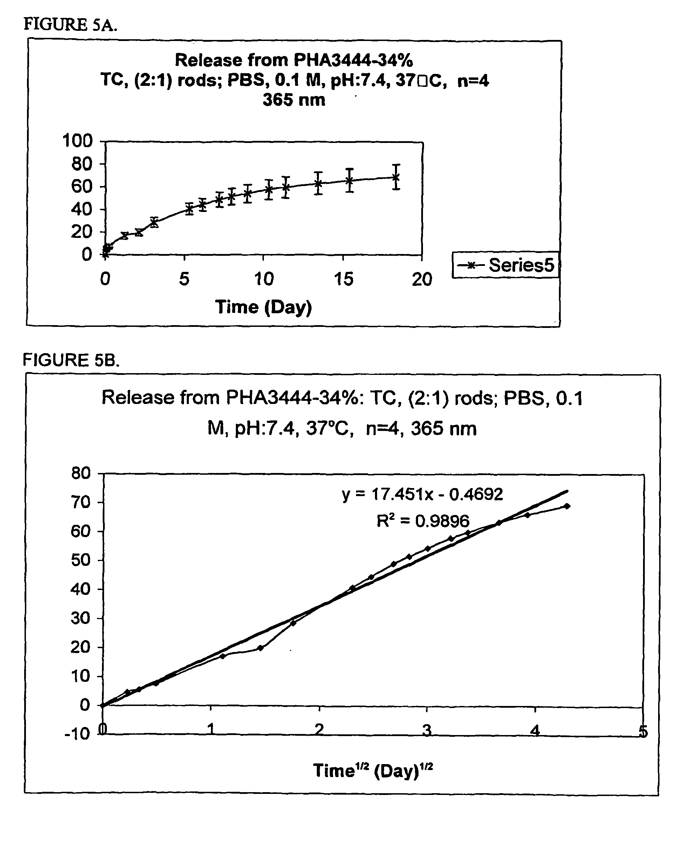

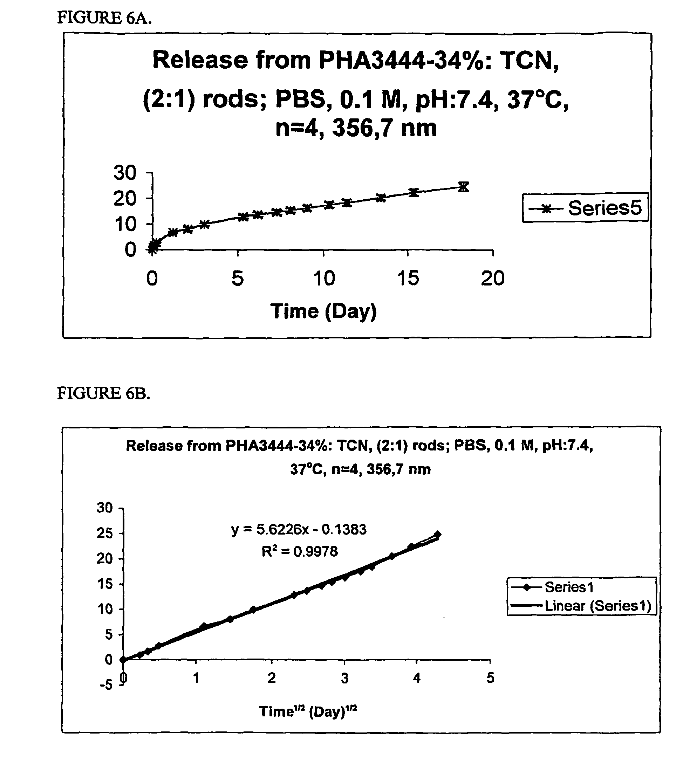

The release behavior appeared to follow Higuchi release kinetics (the k values for TC and TCN were 17.45 and 5.62, respectively) for an 18-day period releasing only a traction of the total content, see Figures 5A and 5B and 6A and 6B. TC released at a higher rate than the less water soluble TCN. The average cumulative release of TC at 17 days was approximately 65% versus 23% for. TCN. No burst of release was observed with either TC or TCN.

Similar results were obtained with PHA3444-50% (PHA3444 containing 50% 44 monomer) PHA polymer loaded 2:1, however, with a short burst releasing almost 25% of the drug. A total of 60% of the TC is released in 15 days, 62% in 23 days, with the release versus time square root plot yielding a straight line as expected from a monolithic release device. Results were not greatly different using a PHA3444-23% (PHA3444 containing 23% 44 monomer) loaded 2:1 with TC or TCN

### EXAMPLE 5: biological Effectiveness of Released Antibiotic

In this Example, the antibiotic properties of the Tetracycline released from the PHA rods was determined in an *in vitro* biological assay against E. coli DH5α. For this *in vitro* bioassay, the Agar Diffusion Method was used and the size of a zone of clearing was determined after applying the antibiotic solution to a petri dish grown with a lawn of *E*. *Coli* DH5α. All the steps of this procedure were carried out under aseptic conditions.

Penassay Broth Medium was prepared using the components in Table 1. The medium pH was. 7.00 ± 0.05 and the sterilization conditions were 121 °C for 15 min. For solid media, agar (1% w/v) was added prior to sterilization. The bacterial strain *E*. *coli* DH5α was inoculated to 200 mL broth mediuni, shaken overnight at 37°C at 200 rpm in an orbital shaker. Inoculate 200 mL bacteria to the plates containing solid Penassay Broth Media.

**Table 1: Penassay Broth Medium components**

| COMPONENT | AMOUNT (g/l) |
|---|---|
| Bacto Beef Exact | 1.5 |
| Bacto Yeast Extract | 1.5 |
| Bacto Peptone | 5.0 |
| Bacto Dextrose | 1.0 |
| NaCl | 3.5 |
| K₂HPO₄ | 3.68 |
| KH₂PO₄ | 1.32 |

On the next day, the TC solutions (25 µL), collected at 1^{st}, 7^{th} and 14^{th} days (release product of the last 24 hours) and sterilized by a microfilter, were applied to sterile filter discs. Two discs containing TC solutions were placed onto each plate and maintained at 37°C for 24 hours. The radius of the clearing zone was determined in mm. The results are shown in Table 2. The results for clearing zones for the tetracycline released from rod made of the PHA3444-23% and PHA3444-50% polymers were similar to that of the PHA3444-34% polymer, but are not shown in Table 2
Negative Control: Applied 25 microliter buffet containing no drug onto the Petri plate
Positive Control: Applied 25 microliter buffer containing 10 mg TC/mL onto the Petri plate
Polymers tested include PHA4400 and PHA3444-34%. The ratio of polymer to Tetracycline antibiotic in the test sample rods is provided below each polymer sample.

**Table 2. Results of the Antibiogram Test**

| Time (days) | Radius of Zone (mm) | | | | |
|---|---|---|---|---|---|
| | ***PHA4400*** 10:1 | PHA4400 5:1 | PHA3444-34% 2:1 | Pos Cont | Neg Cont |
| 1 | 10 | 12 | 15 | 20 | 0 |
| 7 | 9 | 11 | 12 | | |
| 14 | 6 | 6 | 10 | | |

## Claims

1. A biodegradable controlled release drug delivery device comprising drug uniformly distributed in poly-4-hydroxybutyrate homopolymer, wherein the drug is incorporated into the polymer at a percent loading of up to 50% by weight of the device and less than 60% of the drug is released *in vitro* in 0.1 M, pH 7.4, phosphate buffer at 37°C after 10 days.

2. The drug delivery device of claim 1 wherein less than 35% of the drug is released *in vitro* in 0.1 M, pH 7.4, phosphate buffer at 37°C after 10 days.

3. The drug delivery device of claim 1 wherein the drug is selected from the group consisting of proteins, peptides, polysaccharides, nucleic acid molecules, and synthetic or natural organic compounds.

4. The drug delivery device of claim 3 wherein the drug is an antibiotic.

5. The drug delivery device of claim 1 wherein the drug is coated onto a medical device.

6. The drug delivery device of claim 1 wherein the poly-4-hydroxybutyrate is formed into or coated onto a stent.

7. The drug delivery device of claim 1 in a form selected from the group consisting of granules, sheets, films, particles, and molded forms.

8. The drug delivery device of claim 1, wherein the device exhibits linear release or zero-order release of the drug.

9. The drug delivery device of claim 1 wherein the device is manufactured by solvent casting, spray drying or melt extrusion.

10. The drug delivery device of claim 1 wherein the device does not release a burst of drug.

11. The drug delivery device of claim 1 wherein the device releases drug for at least 21 days, at least one month, at least three months or at least six months.

12. A device as defined in any of claims 1-11 for use in drug delivery.

13. The drug delivery device of any preceding claim wherein the drug is selected from bioactive peptides or proteins, such as growth factors, hormones, and cell attachment factors, anti-proliferative agents, antibiotics, chemotherapeutics, anesthetics, small drug molecules, steroids, enzymes, lipids, antigens, antibodies, surfactants, vitamins, flavouring agents, radioactive molecules, sweeteners, nutritional agents, and fragrances.

## Patentansprüche

1. Biologisch abbaubare Arzneistoffzuführungsvorrichtung mit kontrollierter Freisetzung, die einen gleichförmig in einem Poly-4-hydroxybutyrathomopolymer verteilten Arzneistoff umfasst, wobei der Arzneistoff in das Polymer mit einer prozentualen Beladung von bis zu 50 % Gew.-% der Vorrichtung eingearbeitet ist und weniger als 60 % des Arzneistoffs in vitro nach 10 Tagen in einem 0,1 M Phosphatpuffer eines pH-Werts von 7,4 bei 37°C freigesetzt sind.

2. Arzneistoffzuführungsvorrichtung nach Anspruch 1, wobei weniger als 35 % des Arzneistoffs in vitro nach 10 Tagen in einem 0,1 M Phosphatpuffer eines pH-Werts von 7,4 bei 37°C freigesetzt sind.

3. Arzneistoffzuführungsvorrichtung nach Anspruch 1, wobei der Arzneistoff aus der Gruppe von Proteinen, Peptiden, Polysacchariden, Nucleinsäuremolekülen und synthetischen oder natürlich vorkommenden organischen Verbindungen ausgewählt ist.

4. Arzneistoffzuführungsvorrichtung nach Anspruch 3, wobei der Arzneistoff ein Antibiotikum ist.

5. Arzneistoffzuführungsvorrichtung nach Anspruch 1, wobei der Arzneistoff auf eine medizinische Vorrichtung aufgetragen ist.

6. Arzneistoffzuführungsvorrichtung nach Anspruch 1, wobei das Poly-4-hydroxybutyrat in einen Stent eingeformt oder auf einen Stent aufgetragen ist.

7. Arzneistoffzuführungsvorrichtung nach Anspruch 1 in einer Form, die aus der Gruppe von Granulatkörnern, Lagen, Filmen, Teilchen und Formteilen ausgewählt ist.

8. Arzneistoffzuführungsvorrichtung nach Anspruch 1, wobei die Vorrichtung eine lineare Freisetzung oder eine Freisetzung nullter Ordnung für den Arzneistoff zeigt.

9. Arzneistoffzuführungsvorrichtung nach Anspruch 1, wobei die Vorrichtung durch Lösemittelgießen, Sprühtrocknen oder Schmelzextrusion gefertigt wird.

10. Arzneistoffzuführungsvorrichtung nach Anspruch 1, wobei die Vorrichtung den Arzneistoff nicht schlagartig freisetzt.

11. Arzneistoffzuführungsvorrichtung nach Anspruch 1, wobei die Vorrichtung den Arzneistoff über mindestens 21 Tage, mindestens einen Monat, mindestens drei Monate oder mindestens sechs Monate freisetzt.

12. Vorrichtung gemäß der Definition in einem der Ansprüche 1-11 zur Verwendung zur Arzneistoffzuführung.

13. Arzneistoffzuführungsvorrichtung nach einem der vorhergehenden Ansprüche, wobei der Arzneistoff aus biologisch aktiven Peptiden oder Proteinen, beispielsweise Wachstumsfaktoren, Hormonen und Zellkontaktfaktoren, antiproliferativen Mitteln, Antibiotika, Chemotherapeutika, Anästhetika, kleinen Arzneistoffmolekülen, Steroiden, Enzymen, Lipiden, Antigenen, Antikörpern, grenzflächenaktiven Mitteln, Vitaminen, Aromatisierungsmitteln, radioaktiven Molekülen, Süßungsmitteln, Nährstoffen und Duftstoffen ausgewählt ist.

## Revendications

1. Dispositif d'administration de médicament biodégradable à libération prolongée comprenant un médicament distribué uniformément dans un homopolymère de poly(4-hydroxybutyrate), dans lequel le médicament est incorporé dans le polymère à un pourcentage de charge jusqu'à 50% en masse du dispositif et moins de 60% du médicament est libéré *in vitro* dans un tampon phosphate à 0,1 M, pH 7,4 à 37°C après 10 jours.

2. Dispositif d'administration de médicament selon la revendication 1, dans lequel moins de 35% du médicament est libéré *in vitro* dans un tampon phosphate à 0,1 M, pH 7,4 à 37°C après 10 jours.

3. Dispositif d'administration de médicament selon la revendication 1, dans lequel le médicament est choisi dans le groupe constitué par des protéines, des peptides, des polysaccharides, des molécules d'acide nucléique et des composés organiques synthétiques ou naturels.

4. Dispositif d'administration de médicament selon la revendication 3, dans lequel le médicament est un antibiotique.

5. Dispositif d'administration de médicament selon la revendication 1, dans lequel le médicament est revêtu sur un dispositif médical.

6. Dispositif d'administration de médicament selon la revendication 1, dans lequel le poly(4-hydroxybutyrate) est formé dans ou revêtu sur une endoprothèse.

7. Dispositif d'administration de médicament selon la revendication 1, dans une forme choisie dans le groupe constitué par les granules, les feuilles, les films, les particules, et les formes moulées.

8. Dispositif d'administration de médicament selon la revendication 1, dans lequel le dispositif présente une libération linéaire ou une libération d'ordre zéro du médicament.

9. Dispositif d'administration de médicament selon la revendication 1, dans lequel le dispositif est fabriqué par coulage au solvant, séchage par pulvérisation ou extrusion de matière fondue.

10. Dispositif d'administration de médicament selon la revendication 1, dans lequel le dispositif ne libère pas une bouffée de médicament.

11. Dispositif d'administration de médicament selon la revendication 1, dans lequel le dispositif libère le médicament pendant au moins 21 jours, au moins un mois, au moins trois mois ou au moins six mois.

12. Dispositif selon l'une quelconque des revendications 1 à 11, destiné à être utilisé dans l'administration de médicament.

13. Dispositif d'administration de médicament selon l'une quelconque des revendications précédentes, dans lequel le médicament est choisi parmi des peptides ou protéines bioactifs, tels que les facteurs de croissance, les hormones et les facteurs de fixation de cellule, des agents antiprolifératifs, des antibiotiques, des médicaments chimiothérapeutiques, des anesthésiants, des petites molécules de médicament, des stéroïdes, des enzymes, les lipides, des antigènes, des anticorps, des tensioactifs, des vitamines, des agents aromatisants, des molécules radioactives, des édulcorants, des agents nutritionnels et des parfums.
